# FASCICULE DE BREVET EUROPEEN

(11) **EP 2 361 239 B1**
(45) Date de publication et mention de la délivrance du brevet: **28.06.2017**
(21) Numéro de dépôt: 09795467.1
(22) Date de dépôt: 19.11.2009
(51) Int. Cl.: C07C 45/52, C07C 47/22, C07C 319/18, C07C 319/20, C07C 323/22, C07C 323/52, C07C 323/58, C07D 233/76, A23K 20/142, A23K 50/75

(54) **PROCÉDÉ DE FABRICATION DE MÉTHYLMERCAPTOPROPIONALDEHYDE ET DE METHIONINE À PARTIR DE MATIÈRES PREMIÈRES RENOUVELABLES**
VERFAHREN ZUR HERSTELUNG VON METHYLMERCAPTOPROPIONALDEHYD UND METHIONIN UNTER VERWENDUNG ERNEUERBARER ROHSTOFFE
METHOD FOR MANUFACTURING METHYLMERCAPTOPROPIONALDEHYDE AND METHIONINE USING RENEWABLE RAW MATERIALS

(30) Priorité: 20.11.2008 FR 0857886
(43) Date de publication de la demande: 31.08.2011
(73) Titulaire: Arkema France, 92700 Colombes (FR)
(72) Inventeur: DEVAUX, Jean-François, F-69510 Soucieu en Jarrest (FR); DUBOIS, Jean-Luc, F-69390 Millery (FR)
(86) Numéro de dépôt international: PCT/FR2009/052220
(87) Numéro de publication internationale: WO 2010/058129

(56) Documents cités:
- EP-A1- 1 408 029
- WO-A2-2006/087083
- WO-A2-2006/087084
- FR-A- 1 526 355
- US-A- 5 770 769
- US-A1- 2008 119 663
- US-A1- 2008 183 019

## Description

### Domaine de l'invention

La présente invention concerne la production de méthionine, protéine de synthèse utilisée comme complément de l'alimentation animale, à partir d'au moins une matière première renouvelable. Plus précisément, l'invention a pour objet un procédé industriel pour produire du méthylmercaptopropionaldéhyde, intermédiaire clé pour la synthèse de la méthionine, et de la méthionine à partir de matières premières renouvelables.

La méthionine, ou acide 2-amino-4-(méthylthio)butyrique, de formule chimique H₃C-S-(CH₂)₂-CH(NH₂)-COOH est un acide aminé essentiel, non synthétisé par les animaux, nécessaire comme appoint dans la ration alimentaire, notamment de la volaille dont les besoins en méthionine sont importants. La méthionine obtenue par voie de synthèse chimique s'est imposée comme substitut des apports d'origine naturelle (farines de poisson, tourteaux de soja...) pour l'alimentation animale. Le marché principal est celui des aliments pour la volaille.

Contrairement aux autres acides aminés, la méthionine est assimilable biologiquement aussi bien sous la forme dextrogyre (d ou +) que sous la forme lévogyre (l ou -), ce qui a permis le développement de synthèses chimiques conduisant au produit racémique. Ainsi le marché de la méthionine de synthèse est principalement celui de la dl-méthionine, produit solide couramment désigné par DLM. Il existe également un dérivé liquide de la méthionine, l'α-hydroxy acide, correspondant à l'acide 2-hydroxy-4-(méthylthio)butyrique de formule chimique H₃C-S-(CH₂)₂-CH(OH)-COOH, qui a la particularité d'être transformé *in vivo* en méthionine de façon pratiquement quantitative. Ce produit liquide, disponible commercialement sous forme de solution aqueuse à 88% en masse, est désigné couramment par hydroxyanalogue de la méthionine.

De nombreuses synthèses relatives à la méthionine ou à son dérivé hydroxylé ont été décrites, mais les procédés chimiques exploités industriellement reposent essentiellement sur les mêmes matières premières principales et les mêmes intermédiaires clés, à savoir :
- l'acroléine H₂C=CH-CHO et le méthylmercaptan H₃CSH (MSH) conduisant par réaction au méthylmercaptopropionaldéhyde H₃C-S-CH₂-CH₂-CHO (MMP), désigné aussi par 3-(méthylthio)propanal ou par aldéhyde méthylthiopropionique (AMTP),
- l'acide cyanhydrique (HCN) ou le cyanure de sodium (NaCN), qui après réaction avec le MMP, conduit finalement à la méthionine ou à l'hydroxyanalogue de la méthionine.

On pourra se reporter à l'article des Techniques de l'Ingénieur, traité Génie des Procédés, J 6-410-1 à 9 qui décrit les conditions de mise en oeuvre industrielle des procédés de synthèse de la méthionine passant par le méthylmercaptopropionaldéhyde comme produit intermédiaire.

L'acroléine, utilisée comme matière première pour produire le méthylmercaptopropionaldéhyde, est obtenue principalement par oxydation à l'air du propylène en phase gazeuse sur un catalyseur à base de molybdate de bismuth vers 350°C.

L'acide cyanhydrique, mis en oeuvre pour produire la méthionine, est obtenu par réaction d'ammoniac avec du méthane, du méthanol, ou encore avec un hydrocarbure tel que propane ou propylène, éventuellement en présence d'air et/ou d'oxygène.

Il en résulte que le méthylmercaptopropionaldéhyde et par conséquent la méthionine sont des produits de synthèse fortement dépendants de matières premières d'origine fossile ou pétrolière qui contribuent à l'augmentation de l'effet de serre. En effet, le propylène est obtenu par vapocraquage ou craquage catalytique de coupes pétrolières. L'ammoniac est obtenu par réaction de l'azote de l'air et d'hydrogène provenant de reformage par la vapeur d'eau des hydrocarbures présents dans le naphta ou dans le gaz naturel. Le méthane est le composant principal du gaz naturel, combustible fossile constitué d'un mélange d'hydrocarbures présent naturellement dans des roches poreuses sous forme gazeuse. Le propane est extrait soit du pétrole brut au cours des opérations de raffinage, soit du gaz naturel et des gaz associés dans les gisements de pétrole.

Étant donnée la diminution des réserves pétrolières mondiales, la source de ces matières premières va peu à peu s'épuiser. Les matières premières d'origine végétale ou animale sont de source renouvelable et ont un impact réduit sur l'environnement. Elles ne nécessitent pas toutes les étapes de raffinage (très coûteuses en énergie) des produits pétroliers. Elles contribuent d'autant moins au réchauffement climatique que du CO₂ atmosphérique a été consommé par photosynthèse lors de leur biosynthèse.

Il apparaît donc nécessaire de disposer de procédés de synthèse non dépendants de matière première d'origine fossile, mais utilisant plutôt des matières premières d'origine renouvelable.

La présente invention a donc pour but de fournir un procédé de synthèse du méthylmercaptopropionaldéhyde et un procédé de synthèse de la méthionine basé sur l'utilisation de matières premières renouvelables. En particulier, l'acroléine entrant comme matière première dans ces procédés, peut être obtenue comme produit de déshydratation du glycérol, lui-même issu de la méthanolyse des huiles végétales en même temps que les esters méthyliques qui sont employés notamment comme carburants ou combustibles dans le gazole et le fioul domestique.

Le procédé selon l'invention permet ainsi de s'affranchir de la consommation de pétrole, de réduire la consommation d'énergie, et de faire appel à des produits naturels disponibles en grande quantité.

### Technique antérieure

Il est connu depuis longtemps que le glycérol peut conduire à l'obtention d'acroléine.

Le brevet US 5,387,720 décrit un procédé de production d'acroléine par déshydratation du glycérol, en phase liquide ou en phase gaz sur des catalyseurs solides acides définis par leur acidité de Hammett. Selon les auteurs de ce brevet, la réaction en phase gaz est préférable car elle permet d'avoir un taux de conversion du glycérol proche de 100%. Cependant, elle conduit à une solution aqueuse d'acroléine contenant des produits secondaires, tel que l'hydroxypropanone, en quantité importante.

Les demandes WO 2006/087084 et WO 2006/087083 proposent des améliorations à ce procédé par le choix de catalyseurs très acides, ou par l'apport d'oxygène au cours de la réaction de déshydratation du glycérol. Du propionaldéhyde et de l'acétaldéhyde en quantité importante sont co-produits dans ces procédés.

La demande US 2008/0119663 décrit la préparation d'acroléine à partir de triglycérides, la solution aqueuse d'acroléine obtenue pouvant être utilisée comme matière première pour la préparation de méthionine.

Dans aucun de ces documents, il n'est décrit comment purifier l'acroléine produite pour alimenter directement une unité de production de méthylmercaptopropionaldéhyde par réaction avec le méthylmercaptan, ou pour alimenter une unité de production de méthionine.

La réaction entre l'acroléine et le méthylmercaptan (MSH) pour former le méthylmercaptopropionaldéhyde (MMP) peut s'effectuer en phase liquide, après production, purification et isolement sous forme liquide de l'acroléine. La réaction est réalisée en général en présence d'un catalyseur basique, en l'absence de solvant vers 40-50°C, avec un léger excès de MSH. L'acroléine utilisée est généralement de l'acroléine commerciale contenant quelques % d'eau. Des catalyseurs utilisables pour la réaction en phase liquide sont par exemple des bases organiques, éventuellement combinées avec un acide organique. On peut citer par exemple les bases organiques sélectionnées parmi des amines telles que celles décrites dans la demande WO 1996/40631, les bases organiques de type N-alkylmorpholine décrites dans le document EP 1 556 343, ou les systèmes constitués d'un composé basique et d'un composé acide dans des rapports molaires inférieurs à 0,3/1 décrits dans le document EP 1 408 029. Le catalyseur le plus classique est la pyridine associée à l'acide acétique. Les rendements sont pratiquement quantitatifs. Le MMP obtenu est ensuite purifié par distillation. On sépare ainsi des impuretés légères et des produits lourds soufrés qui sont ensuite incinérés.

De nombreux documents décrivent des procédés de mise en oeuvre de la réaction entre l'acroléine liquide et le méthylmercaptan. On peut citer le document FR 1 526 355 qui décrit la synthèse de MMP par réaction de MSH avec une solution aqueuse d'acroléine contenant au moins un composé carbonylé choisi parmi les aldéhydes saturés aliphatiques et les cétones aliphatiques saturées mais exempte d'acides organiques insaturés. La solution aqueuse d'acroléine est obtenue à partir de propylène ou par condensation de formaldéhyde avec l'acétaldéhyde, et purification partielle par élimination des composants ayant un point d'ébullition supérieur à celui de l'acroléine. Les sous-produits présents dans la solution d'acroléine, principalement acétaldéhyde, propionaldéhyde et acétone, doivent ensuite être séparés au cours de la purification du MMP après réaction avec le MSH.

Dans le document FR 1 520 328, il a été proposé d'effectuer la synthèse du MMP à partir d'acroléine en deux étapes, consistant à mettre en contact du MSH avec du MMP jusqu'à ce que le dégagement de chaleur produit soit terminé, puis à mettre en contact le produit réactionnel résultant avec de l'acroléine. Ce procédé permet un contrôle efficace de la température de réaction.

Le MMP peut être obtenu aussi par synthèse directe par réaction de méthylmercaptan avec l'effluent gazeux contenant de l'acroléine provenant de l'oxydation catalytique du propylène, après purification partielle de ce flux, en utilisant le MMP lui-même à la fois comme solvant d'absorption de l'acroléine et comme réactif avec le MSH (FR 2 314 917, EP 022 697, EP 889 029, WO 94/29254, WO 97/00858).

Dans les documents FR 2 314 917 et EP 022 697, le mélange gazeux contenant l'acroléine est débarrassé de l'acide acrylique et de l'eau qu'il contient par condensation à basse température, avant réaction avec le méthylmercaptan. Une absorption à l'aide de MMP du flux gazeux ainsi purifié peut être mise en oeuvre préalablement à la réaction avec le méthylmercaptan.

Dans les documents WO 94/29254 et WO 97/00858, l'effluent gazeux contenant l'acroléine, de la vapeur d'eau et des gaz incondensables est mis en contact avec un milieu réactionnel contenant du MMP, du méthylmercaptan et un catalyseur de réaction entre l'acroléine et le méthylmercaptan, pour former un produit de réaction liquide contenant le MMP. Le rapport molaire entre la vapeur d'eau et l'acroléine présentes dans l'effluent gazeux est de préférence inférieur à 0,3. Les gaz incondensables incluant l'oxygène, l'azote, le monoxyde de carbone et le dioxyde de carbone, sont présents dans l'effluent gazeux dans une proportion pouvant atteindre de 60% à 80%, ils doivent être ensuite séparés du produit de réaction liquide. Ce procédé présente l'inconvénient d'envoyer un flux important de gaz incondensables dans le milieu réactionnel, ce qui peut entraîner une perte de méthylmercaptan de ce milieu lors de leur séparation et générer un flux d'incondensables pollués par des composés soufrés.

Dans le document WO 97/36848, le flux gazeux d'acroléine, issu d'une réaction du propylène avec de l'oxygène en présence de propane comme diluant, est partiellement condensé pour donner un flux d'acroléine contenant de l'acétaldéhyde à une teneur comprise entre 0,5% et 3,5% en poids et de l'eau à une teneur comprise entre 2% et 8%. Ce flux d'acroléine, après réaction avec le méthylmercaptan, conduit à du MMP comprenant une forte teneur en acétaldéhyde qui doit alors être séparé par distillation.

Par ailleurs, des procédés ont été proposés pour traiter et purifier un flux gazeux contenant de l'acroléine issu de l'oxydation du propylène, en vue de sa mise en oeuvre dans une réaction de synthèse du MMP. On peut citer par exemple le document EP 751 110 qui décrit un procédé de purification d'un mélange gazeux comprenant de l'acroléine, de l'eau, des acides (acide acrylique, acide formique, acide acétique, acide maléique) et des incondensables (N₂, O₂, autres gaz de l'air, CO, CO₂, propylène), ce procédé minimisant les risques de dégradation de l'acroléine tout en évitant l'encrassement des appareillages mis en oeuvre dans le procédé. Ce procédé repose essentiellement sur une condensation à basse température de l'eau et de l'essentiel des acides présents dans le flux, et d'un stripage des phases aqueuses condensées pour récupérer l'acroléine purifiée. On obtient une fraction gazeuse purifiée contenant de l'acroléine et des incondensables dont la teneur pondérale en eau est inférieure à 2% et la teneur pondérale en acides est inférieure à 100 ppm. Cette solution technique présente néanmoins l'inconvénient de fournir un flux gazeux d'acroléine contenant des gaz incondensables en quantité importante, ce qui, après réaction avec le méthylmercaptan, conduit à un flux important de composés incondensables pollués par des composés soufrés. La purification du MMP produit peut s'avérer alors difficile.

Il existe aussi des procédés de synthèse de MMP complètement intégrés au départ de propylène, sans isolement de l'acroléine intermédiaire, ce qui réduit les risques liés au stockage et à la manipulation d'acroléine. Ces procédés nécessitent néanmoins différentes étapes d'absorption et de stripage du flux gazeux dérivé de l'oxydation du propylène. Dans le document WO 03/068721, les gaz incondensables présents dans le produit brut d'oxydation catalytique du propylène sont séparés en amont de la réaction avec le méthylmercaptan, et ainsi peuvent être recyclés vers l'oxydation du propylène, sans avoir été pollués par aucun soufré généré par le méthylmercaptan ou le MMP. Le flux gazeux d'acroléine issu des étapes d'absorption et stripage contient de l'eau mais peut être utilisé directement sous forme gazeuse pour la synthèse du MMP. Ce procédé reste cependant dépendant d'une matière première d'origine fossile.

Plus récemment, la demande US 2006/0183945 décrit la synthèse directe de MMP à partir de glycérol. Le procédé consiste à mettre en contact du glycérol avec du méthylmercaptan, en phase liquide ou en phase gaz, en présence d'un catalyseur solide acide et éventuellement d'un solvant. La réaction en phase liquide est réalisée à une température comprise entre 50°C et 500°C sous une pression allant de 1 à 300 bars et un solvant ou un diluant tel que de l'eau, un alcool, de l'acétone, du toluène, ou le MMP lui-même. La concentration en glycérol est de 1% à 100%, de préférence de 5% à 40% par rapport au solvant ou diluant. Le rapport molaire entre le glycérol et le MSH est ajusté entre 0,2 et 50, de préférence entre 0,8 et 10. Lorsque la synthèse est réalisée en phase gazeuse, la température de réaction est comprise entre 200°C et 550°C, en particulier entre 250°C et 350°C, sous une pression allant de 1 à 100 bars, de préférence de 1 à 30 bars. Le mélange gazeux peut être dilué par exemple dans de l'azote, de l'air ou de la vapeur d'eau. Les catalyseurs utilisables sont des catalyseurs hétérogènes acides présentant une acidité de Hammett inférieure à +2, de préférence inférieure à -3. Le méthylmercaptan peut ne pas être ajouté immédiatement au début de la réaction, mais après une conversion partielle du glycérol dans les conditions de mise en oeuvre de la réaction. Le procédé est mis en oeuvre en batch, ou en semi-continu avec introduction continue du méthylmercaptan, dans un réacteur à une seule zone, ou un réacteur à deux zones réactionnelles. Les rendements en MMP obtenus à partir de ce procédé sont selon les exemples de 29% et 4% molaires par rapport au glycérol. Ces rendements sont insuffisants pour envisager une production industrielle de MMP sur la base de ce procédé. Par ailleurs de tels rendements obligeraient à une surconsommation d'énergie et diminueraient considérablement l'intérêt environemental d'un procédé de production à partir d'une matière première renouvelable.

La demande US 2008/0183019 décrit également un procédé de production de MMP à partir de glycérol et de MSH directement en une étape en utilisant un catalyseur présentant une acidité de Hammett inférieure à +2 et qui comprend du tungstène et un ou plusieurs promoteurs. Le fait de procéder en une seule étape nécessite d'effectuer la réaction dans des zones de températures où le MMP est peu stable, ce qui limite fortement les rendements.

S'agissant de la méthionine, elle est obtenue par réaction du MMP, soit avec du cyanure de sodium (NaCN) en présence de CO₂ et NH₃, puis saponification par une base forte de la solution aqueuse d'hydantoïne formée puis acidification (synthèse de Bücherer), soit avec de l'acide cyanhydrique (HCN) pour former la cyanhydrine intermédiaire qui est ensuite transformée en aminonitrile, puis hydrolysé en méthionine (synthèse de Strecker).

Une variante de ces procédés réside dans la synthèse de l'hydantoïne intermédiaire à partir de MMP et de HCN, puis saponification au carbonate de potassium et acidification (procédé Degussa), décrite par exemple dans le brevet US 5,990,349.

La synthèse de l'hydroxyanalogue de la méthionine passe aussi par la synthèse de la cyanhydrine intermédiaire, qui est ensuite hydrolysée en deux étapes, la première étape s'effectuant à basse température pour former l'amide, la seconde étape à plus forte température conduisant au produit recherché en l'absence de sous-produits lourds.

On peut citer les documents suivants : US 2,745,745 ; EP 142 488 et EP 333 527 qui décrivent des procédés de production d'hydroxyanalogue de la méthionine.

Plus récemment, dans la demande internationale WO 2008/002053, il est proposé un procédé de production d'amino-acides, telle que par exemple la méthionine, par fermentation de glycérol.

### Résumé de l'invention

La Société Déposante a maintenant trouvé un procédé de fabrication du méthylmercaptopropionaldéhyde par addition de méthylmercaptan sur l'acroléine, caractérisé par le fait qu'au moins l'un parmi l'acroléine et le méthylmercaptan mis en jeu dans cette réaction a été obtenu par une réaction ou une succession de réactions à partir de la biomasse, ledit procédé comprenant au moins les étapes suivantes :
(a) la déshydratation de glycérol en acroléine à partir d'une solution aqueuse de glycérol en présence d'un catalyseur acide,
(b) la purification du flux aqueux issu de l'étape (a) pour obtenir un flux d'acroléine contenant moins de 15%, de préférence moins de 7%, de préférence encore moins de 4% massique d'eau par rapport à l'acroléine,
(c) la réaction du flux d'acroléine obtenu à l'étape (b) avec du méthylmercaptan en présence d'un catalyseur,
(d) éventuellement, la purification du produit obtenu à l'étape (c).

L'invention aussi pour objet un procédé de fabrication de la méthionine ou de l'hydroxyanalogue de la méthionine par réaction de l'acide cyanhydrique ou du sel de sodium de l'acide cyanhydrique sur le méthylmercaptopropionaldéhyde, le méthylmercaptopropionaldéhyde ayant été obtenu par addition de méthylmercaptan sur l'acroléine, caractérisé par le fait qu'au moins l'un parmi l'acroléine, le méthylmercaptan ou l'acide cyanhydrique mis en jeu a été obtenu par une réaction ou une succession de réactions à partir de la biomasse, ledit procédé comprenant au moins les étapes suivantes :
(a) la déshydratation de glycérol en acroléine à partir d'une solution aqueuse de glycérol en présence d'un catalyseur acide,
(b) la purification du flux aqueux issu de l'étape (a) pour obtenir un flux d'acroléine contenant moins de 15%, de préférence moins de 7%, de préférence encore moins de 4% massique d'eau par rapport à l'acroléine,
(c) la réaction du flux d'acroléine obtenu à l'étape (b) avec du méthylmercaptan en présence d'un catalyseur,
(d) éventuellement, la purification du produit obtenu à l'étape (c),
(e) la réaction du produit obtenu à l'étape (c) ou (d) avec de l'acide cyanhydrique, ou du cyanure de sodium,
(f) la transformation du produit obtenu en (e) en méthionine ou hydroxyanalogue de la méthionine et sa purification.

L'invention a pour effet de réduire le réchauffement de la planète lors de la fabrication de MMP et de méthionine, en réduisant les émissions de gaz à effet de serre liées à leur fabrication. Par ailleurs, l'utilisation de matières premières renouvelables pour l'ensemble des composés mis en oeuvre dans les procédés selon l'invention permettra de consolider la nature « bio » de ces procédés, les produits obtenus, MMP et méthionine pouvant alors être constitués de 100% de carbone organique issu de ressources renouvelables.

### Exposé détaillé de l'invention

L'invention utilise comme matière première la biomasse. On entend par biomasse la matière première d'origine végétale ou animale produite naturellement. Cette matière végétale se caractérise par le fait que la plante pour sa croissance a consommé du CO₂ atmosphérique tout en produisant de l'oxygène. Les animaux pour leur croissance ont de leur côté consommé cette matière première végétale et ont ainsi assimilé le carbone dérivé du CO₂ atmosphérique.

La présente invention répond ainsi à certaines préoccupations de développement durable, par l'obtention de méthylmercaptopropionaldéhyde et de méthionine ayant au moins une partie de leurs carbones d'origine renouvelable.

Une matière première renouvelable est une ressource naturelle, animale ou végétale, dont le stock peut se reconstituer sur une période courte à l'échelle humaine. Il faut en particulier que ce stock puisse se renouveler aussi vite qu'il est consommé.

À la différence des matériaux issus de matières fossiles, les matières premières renouvelables contiennent du ¹⁴C dans les mêmes proportions que le CO₂ atmosphérique. Tous les échantillons de carbone tirés d'organismes vivants (animaux ou végétaux) sont en fait un mélange de 3 isotopes : ¹²C (représentant environ 98,892 %), ¹³C (environ 1,108 %) et ¹⁴C (traces 1,2.10⁻¹⁰ %). Le rapport ¹⁴C/¹²C des tissus vivants est identique à celui de l'atmosphère. Dans l'environnement, le ¹⁴C existe sous deux formes prépondérantes : sous forme minérale, c'est-à-dire de gaz carbonique (CO₂), et sous forme organique, c'est-à-dire de carbone intégré dans des molécules organiques.

Dans un organisme vivant, le rapport ¹⁴C/¹²C est maintenu constant par le métabolisme car le carbone est continuellement échangé avec l'environnement. La proportion de ¹⁴C étant constante dans l'atmosphère, il en est de même dans l'organisme, tant qu'il est vivant, puisqu'il absorbe ce ¹⁴C comme il absorbe le ¹²C. Le rapport moyen de ¹⁴C/¹²C est égal à 1,2 x 10⁻¹², pour une matière d'origine renouvelable, tandis qu'une matière première fossile a un rapport nul. Le carbone 14 est issu du bombardement de l'azote atmosphérique (14), et s'oxyde spontanément avec l'oxygène de l'air pour donner le CO₂. Dans notre histoire humaine, la teneur en ¹⁴CO₂ a augmenté à la suite des explosions nucléaires atmosphériques, puis n'a cessé de décroître après l'arrêt de ces essais.

Le ¹²C est stable, c'est-à-dire que le nombre d'atomes de ¹²C dans un échantillon donné est constant au cours du temps. Le ¹⁴C, lui, est radioactif (chaque gramme de carbone d'un être vivant contient suffisamment d'isotopes ¹⁴C pour donner 13,6 désintégrations par minute) et le nombre de tels atomes dans un échantillon décroît au cours du temps (t) selon la loi : n = no exp(-at), dans laquelle:
- no est le nombre de ¹⁴C à l'origine (à la mort de la créature, animal ou plante),
- n est le nombre d'atomes ¹⁴C restant au bout du temps t,
- a est la constante de désintégration (ou constante radioactive) ; elle est reliée à la demi-vie.

La demi-vie (ou période) est la durée au bout de laquelle un nombre quelconque de noyaux radioactifs ou de particules instables d'une espèce donnée, est réduit de moitié par désintégration ; la demi-vie T_{1/2} est reliée à la constante de désintégration a par la formule aT_{1/2}= In 2. La demi-vie du ¹⁴C vaut 5730 ans. En 50 000 ans la teneur en ¹⁴C est inférieure à 0,2 % de la teneur initiale et devient donc difficilement décelable. Les produits pétroliers, ou le gaz naturel ou encore le charbon ne contiennent donc pas de ¹⁴C.

Compte tenu de la demi-vie (T_{1/2}) du ¹⁴C, la teneur en ¹⁴C est sensiblement constante depuis l'extraction des matières premières renouvelables, jusqu'à la fabrication des « biomatériaux » issus de ces matières premières, et même jusqu'à la fin de leur utilisation.

La teneur en ¹⁴C d'un matériau d'origine renouvelable peut être mesurée par exemple selon les techniques suivantes :
- par spectrométrie à scintillation liquide : cette méthode consiste à compter des particules 'Bêta' issues de la désintégration du ¹⁴C. On mesure le rayonnement Bêta issu d'un échantillon de masse connue (nombre d'atomes de carbone connu) pendant un certain temps. Cette 'radioactivité' est proportionnelle au nombre d'atomes de ¹⁴C, que l'on peut ainsi déterminer. Le ¹⁴C présent dans l'échantillon émet des rayonnements β-, qui, au contact du liquide scintillant (scintillateur), donnent naissance à des photons. Ces photons ont des énergies différentes (comprises entre 0 et 156 KeV) et forment ce que l'on appelle un spectre de ¹⁴C. Selon deux variantes de cette méthode, l'analyse porte soit sur le CO₂ préalablement produit par combustion de l'échantillon carboné dans une solution absorbante appropriée, soit sur le benzène après conversion préalable de l'échantillon carboné en benzène.
- par spectrométrie de masse : l'échantillon est réduit en graphite ou en CO₂ gazeux, analysé dans un spectromètre de masse. Cette technique utilise un accélérateur et un spectromètre de masse pour séparer les ions ¹⁴C des ¹²C et donc déterminer le rapport des deux isotopes.

Ces méthodes de mesure de la teneur en ¹⁴C des matériaux sont décrites précisément dans les normes ASTM D 6866 (notamment D6866-06) et dans les normes ASTMD 7026 (notamment 7026-04). Ces méthodes comparent les données mesurées sur l'échantillon analysé avec les données d'un échantillon référence d'origine 100% renouvelable, pour donner un pourcentage relatif de carbone d'origine renouvelable dans l'échantillon.

La méthode de mesure préférentiellement utilisée est la spectrométrie de masse décrite dans la norme ASTM D6866-06 (« accelerator mass spectroscopy »).

Dans la présente invention, le méthylmercaptopropionaldéhyde et la méthionine proviennent au moins en partie de biomasse, ils contiennent du carbone organique issu de matières premières renouvelables et sont de ce fait caractérisés en ce qu'ils contiennent du ¹⁴C. En particulier, au moins 25%, de préférence au moins 50% en masse des carbones du MMP est d'origine renouvelable et au moins 20%, de préférence au moins 50% en masse des carbones de la méthionine est d'origine renouvelable. Le MMP et la méthionine peuvent comprendre 100% de carbone d'origine renouvelable, lorsque toutes les matières premières mises en oeuvre sont d'origine renouvelable.

Conformément à un mode de réalisation préféré de l'invention, on a obtenu l'acroléine à partir de glycérol. Le glycérol est obtenu comme sous-produit de la fabrication de biocarburants ou de savons ou d'alcool ou d'acides ou esters gras à partir de plantes oléagineuses comme le colza, le tournesol, le soja, le palmier à huile, le jatropha, le ricin, ou à partir de graisses animales.

Conformément à un mode de réalisation, on a obtenu le méthyl mercaptan à partir d'hydrogène sulfuré H₂S et de méthanol en présence d'un catalyseur selon la réaction : 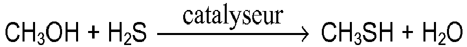 le méthanol pouvant dériver de la pyrolyse du bois, ou de la fermentation de biomasse, par exemple de cultures de plantes comme le blé, la canne à sucre ou la betterave donnant des produits fermentables.

Le méthanol peut aussi provenir de la gazéification de toutes matières d'origine animale ou végétale conduisant à un gaz de synthèse composé essentiellement de monoxyde de carbone et d'hydrogène que l'on fait réagir avec l'eau. Les matières d'origine animale sont à titre d'exemples non limitatifs les huiles et graisses de poisson, telles que huile de foie de morue, huile de baleine, de cachalot, de dauphin, de phoque, de sardine, de hareng, de squales, les huiles et graisses de bovins, porcins, caprins, équidés, et volailles, telles que suif, saindoux, graisse de lait, lard, graisses de poulet, de boeuf, de porc, de cheval, et autres. Les matières d'origine végétale sont par exemple les huiles végétales, le fourrage de paille de céréales, comme paille de blé, paille de maïs ; résidus de céréales, comme résidus de maïs ; farines de céréales, comme farine de blé ; céréales telles que le blé, l'orge, le sorgho, le maïs ; bois, déchets et rebuts de bois ; grains ; canne à sucre, résidus de canne à sucre ; sarments et tiges de pois ; betterave, mélasses telles que mélasses de betteraves ; pommes de terre, fanes de pommes de terre, résidus de pommes de terre ; amidon ; mélanges de cellulose, hémicellulose et lignine ; ou la liqueur noire de papeterie.

Sur la transformation du gaz de synthèse en méthanol, selon la réaction :

CO + 2H₂ → CH₃OH

on peut se référer à "Procédés de pétrochimie, IFP, ENSPM", 1985, 2e édition, pp 90-104 et à "Fundamentals of Industrial Catalytic Processes", Wiley, 2e édition, 6.4.8.

Le méthanol peut aussi résulter de l'oxydation directe (contrôlée) du méthane produit à partir de biomasse :

Biomasse (fermentation) → CH₄ et CH₄ + ½ O₂ → CH₃OH.

La production de méthane à partir de la biomasse est connue. Le méthane est obtenu à partir de biogaz. Le biogaz est le gaz produit par la fermentation de matières organiques animales et/ou végétales en l'absence d'oxygène. Cette fermentation, appelée aussi méthanisation, se produit naturellement ou spontanément dans les décharges contenant des déchets organiques, mais peut être effectuée dans des digesteurs, pour traiter par exemples des boues d'épuration, des déchets organiques industriels ou agricoles, des lisiers de porc, des ordures ménagères. De préférence, on utilise de la biomasse contenant des déjections animales qui servent d'intrant azoté nécessaire à la croissance des microorganismes assurant la fermentation de la biomasse en méthane. Le biogaz est composé essentiellement de méthane et de gaz carbonique, le gaz carbonique est ensuite éliminé par lavage du biogaz à l'aide d'une solution aqueuse basique de soude, potasse ou amine, ou encore par de l'eau sous pression ou par absorption dans un solvant tel que le méthanol. Il est possible d'obtenir selon cette voie du méthane pur de qualité constante. On pourra se référer aux différentes technologies de méthanisation de l'état de la technique, à l'article « Review of Current Status of Anaerobic Digestion Technology for Treatment of Municipal Solid Waste », November 1998, RISE-AT et aux différents procédés biologiques existants pour le traitement des eaux résiduaires, tel que par exemple le procédé Laran^{®} de Linde.

Concernant la méthode de synthèse du méthylmercaptan à partir du méthanol, on pourra se reporter par exemple à la méthode décrite dans les brevets FR 7 343 539 et FR 2 477 538, qui consiste à réaliser la synthèse de MSH par réaction en phase vapeur entre le méthanol et H₂S à une température comprise, en tout point de la masse réactionnelle entre 280°C et 450°C de préférence 320°C et 370°C, sous une pression comprise entre 2,5 et 25 bars de préférence entre 7 et 12 bars, la réaction étant effectuée par passage des réactifs sur au moins trois lits de catalyseur successifs, la totalité de H₂S étant introduite au niveau du premier lit et une fraction du méthanol total étant introduite au niveau de chaque lit, et le rapport molaire global de H₂S au méthanol total étant compris entre 1,10 et 2,5. Le catalyseur utilisé pour cette réaction est de préférence une alumine activée de surface spécifique comprise entre 100 et 400 m²/g telle que sous forme de billes de 2 mm à 5 mm de diamètre.

Selon un mode de réalisation de l'invention, on a obtenu le méthylmercaptan par réaction directe de gaz de synthèse issu de biomasse, composé essentiellement de monoxyde de carbone et d'hydrogène, avec l'hydrogène sulfuré selon un procédé catalytique, et sans passer par le méthanol :

CO + 2H₂ + H₂S → CH₃SH + H₂O

Conformément à un mode de réalisation de l'invention, on a obtenu l'acide cyanhydrique par réaction d'ammoniac sur du méthane ou du méthanol, éventuellement en présence d'air et/ou d'oxygène, l'un au moins des réactifs choisis parmi l'ammoniac, le méthane et le méthanol ayant été obtenu à partir de biomasse.

La production industrielle d'acide cyanhydrique HCN actuelle est basée principalement sur le procédé Andrussow datant des années 1930, consistant en une ammoxydation du méthane suivant laquelle on fait réagir de l'ammoniac avec du méthane en présence d'air et éventuellement d'oxygène sur un catalyseur composé de toiles de platine rhodié à une température allant de 1050°C à 1150°C, selon la réaction :

CH₄ + NH₃ + 3/2 O₂ → HCN + 3 H₂O + chaleur

Généralement, le rapport molaire CH₄/NH₃ va de 1,0 à 1,2, le rapport molaire (CH₄ + NH₃)/O₂ total va de 1,6 à 1,9 ; la pression est généralement de 1 à 2 bars.

Un autre procédé (Degussa) pour produire HCN est basé sur la réaction d'ammoniac avec du méthane, en l'absence d'oxygène ou d'air, à une température de l'ordre de 1300°C. La réaction s'effectue dans des tubes d'alumine frittée revêtus intérieurement de platine. Le faisceau de tubes est chauffé au gaz à l'intérieur d'un four.

On peut utiliser aussi du méthanol en remplacement du méthane pour produire HCN selon la réaction :

CH₃OH + NH₃ + O₂ → HCN + 3 H₂O

Ce procédé, décrit notamment dans les années 1950-1960 dans les brevets GB 718,112 et GB 913,836 de Distillers Company, met en oeuvre un catalyseur à base d'oxyde de molybdène à une température allant de 340°C à 450°C, ou un catalyseur à base d'antimoine et d'étain à une température allant de 350°C à 600°C.

L'ammoniac peut être obtenu à partir d'hydrogène issu d'un gaz de synthèse (composé essentiellement de monoxyde de carbone et d'hydrogène) résultant de la gazéification de la biomasse. La gazéification est un procédé thermochimique permettant de produire un gaz riche en hydrogène à partir de la biomasse et d'un réactif gazeux tel que l'air, l'oxygène ou la vapeur d'eau. La transformation a lieu à haute température (800-1000°C) et généralement à pression atmosphérique ou faible surpression. La concentration en oxygène (dans l'air ou l'eau) n'est pas suffisante lors de la gazéification pour conduire à l'oxydation complète. Ainsi, de grandes quantités de CO et H₂ sont produites selon les réactions suivantes :

C + H₂O →CO + H₂

C + CO₂ → 2 CO

Comme biomasse, on peut utiliser toute matière d'origine animale ou végétale déjà indiquée précédemment.

L'hydrogène, après conversion par la vapeur d'eau du monoxyde de carbone produit du gaz de synthèse est purifié avant d'être introduit dans un réacteur catalytique de synthèse d'ammoniac à haute pression (100 à 250 bars).

L'hydrogène utilisé pour préparer l'ammoniac peut provenir aussi de la récupération de liqueur résiduaire de la fabrication des pâtes cellulosiques. On pourra se référer aux documents FR 2 544 758, EP 666 831 ou US 7,294,225 de Chemrec qui décrivent notamment la gazéification de liqueurs résiduaires de la fabrication de cellulose.

Le méthane, comme déjà indiqué ci-dessus peut être obtenu à partir de biogaz (CH₄/CO₂) produit par la fermentation de matières organiques animales ou végétales en l'absence d'oxygène, le CO₂ étant éliminé par lavage du biogaz à l'aide d'une solution aqueuse basique de soude, potasse ou amine, ou encore par de l'eau sous pression ou par absorption dans un solvant.

Le méthanol, comme déjà indiqué ci-dessus, peut être obtenu par fermentation de biomasse, ou par pyrolyse du bois, ou par gazéification de toutes matières d'origine animale ou végétale conduisant à un gaz de synthèse composé essentiellement de monoxyde de carbone et d'hydrogène que l'on fait réagir avec l'eau, ou par oxydation directe de méthane produit à partir de biomasse.

L'invention a aussi pour objet un procédé de fabrication du méthylmercaptopropionaldéhyde comprenant au moins les étapes suivantes :
(a) la déshydratation de glycérol en acroléine à partir d'une solution aqueuse de glycérol en présence d'un catalyseur acide,
(b) la purification du flux aqueux issu de l'étape (a) pour obtenir un flux d'acroléine contenant moins de 15%, de préférence moins de 7%, de préférence encore moins de 4% massique d'eau par rapport à l'acroléine,
(c) la réaction du flux d'acroléine obtenu à l'étape (b) avec du méthylmercaptan en présence d'un catalyseur,
(d) éventuellement la purification du produit obtenu à l'étape (c).

Le MMP produit selon le procédé de l'invention à l'issue de l'étape (c) ou (d) peut en outre être soumis à une réaction avec de l'acide cyanhydrique, ou du cyanure de sodium au cours d'une étape (e) suivi d'une transformation ultérieure pour produire de la méthionine ou de l'hydroxyanalogue de la méthionine qui peut être alors éventuellement purifié.

La première étape (a) de déshydratation du glycérol peut être effectuée en phase gaz dans un réacteur en présence d'un catalyseur à une température allant de 150°C à 500°C, de préférence comprise entre 250°C et 350°C, et une pression comprise entre 10⁵ et 5.10⁵ Pa.

La déshydratation du glycérol peut être effectuée en phase liquide, dans ce cas la température est comprise entre 150°C et 350°C sous une pression allant de 5.10⁵ Pa à 100.10⁵ Pa.

De préférence, on effectue la première étape en phase gaz.

Le réacteur utilisé peut fonctionner en lit fixe, en lit fluidisé ou en lit fluidisé circulant, ou dans une configuration en modules (plaques ou paniers), en présence de catalyseurs solides acides.

Les catalyseurs qui conviennent sont des matériaux homogènes ou multiphases, insolubles dans le milieu réactionnel qui ont une acidité de Hammett, notée H₀ inférieure à +2 comme indiqué dans le brevet US 5,387,720 qui fait référence à l'article de K. Tanabe et al. dans "Studies in Surface Science and Catalysis", Vol. 51, (1989), chap. 1 et 2 ; l'acidité de Hammett est déterminée par titration amine à l'aide d'indicateurs ou par adsorption d'une base en phase gazeuse. Les catalyseurs répondant au critère d'acidité H₀ inférieur à +2, peuvent être choisis parmi des matériaux siliceux naturels ou de synthèse ou les zéolithes acides ; des supports minéraux, tels que des oxydes, recouverts par des acides inorganiques, mono-, di-, tri- ou poly-acides ; des oxydes ou oxydes mixtes ou encore des hétéropolyacides.

Ces catalyseurs pourront généralement être constitués par un sel d'hétéropolyacide dans lequel des protons dudit hétéropolyacide sont échangés avec au moins un cation choisi parmi les éléments appartenant aux Groupes I à XVI de la Classification Périodique des Eléments, ces sels d'hétéropolyacide contenant au moins un élément choisi parmi le groupe comprenant W, Mo et V.

Parmi les oxydes mixtes, on peut citer également ceux à base de fer et de phosphore et ceux à base de césium, phosphore et tungstène.

Avantageusement, les catalyseurs sont choisis parmi les zéolithes, les composites Nafion^{®} (à base d'acide sulfonique de polymères fluorés), les alumines chlorées, les acides et sels d'acides phosphotungstiques et/ou silicotungstiques, et différents solides de type oxydes métalliques tels que l'oxyde de tantale Ta₂O₅, l'oxyde de niobium Nb₂O₅, l'alumine Al₂O₃, l'oxyde de titane TiO₂, la zircone ZrO₂, l'oxyde d'étain SnO₂, la silice SiO₂ ou silico-aluminate SiO₂-Al₂O₃, imprégnés de fonctions acides telles que borate BO₃, sulfate SO₄, tungstate WO₃, phosphate PO₄, silicate SiO₂, ou molybdate MoO₃. Selon les données de la littérature, ces catalyseurs ont tous une acidité de Hammett H₀ inférieure à +2.

Les catalyseurs préférés sont les zircones sulfatées, les zircones phosphatées, les zircones tungstées, les zircones silicées, les oxydes de titane ou d'étain ou les alumines ou silices phosphatés ou phosphotungstés.

Dans le procédé de l'invention, on part généralement de glycérol brut, c'est à dire contenant typiquement 80-90% de glycérol, 1 à 10% de sels, 1 à 4% de matières organiques non glycérineuses et 3 à 15% d'eau. Avantageusement on part de glycérol dessalé, qui peut être obtenu à partir de glycérol brut par tout moyen connu de l'homme de l'art, comme une distillation sous pression réduite ou un flash sous pression réduite ou une séparation utilisant des résines échangeuses d'ion tel que décrit par exemple dans la demande EP1978009. On peut aussi partir de glycérine sans sel obtenue par des procédés de transestérification d'huiles catalysés par des catalyseurs hétérogènes. On peut aussi partir de glycérine raffinée d'une pureté supérieure à 98%, 99% ou 99,5%.

À l'entrée du réacteur, on utilise généralement un mélange de glycérol et d'eau de concentration massique en glycérol allant de 20% à 99%, de préférence entre 30% et 80%.

Le mélange glycérol/eau peut être utilisé sous forme liquide ou sous forme gazeuse, de préférence sous forme phase gaz.

Un mode de réalisation préféré de l'invention consiste à envoyer un mélange contenant au moins du glycérol, de l'eau, de l'oxygène ou un gaz contenant de l'oxygène, et le cas échéant un gaz inerte et/ou des gaz de recyclage, en phase gaz, sur un lit d'un système catalytique tel que défini précédemment, maintenu à une température de réaction comprise entre 150 et 500°C.

La quantité d'oxygène est choisie de façon à être en dehors du domaine d'inflammabilité en tout point de l'installation. Le rapport molaire entre l'oxygène moléculaire et le glycérol est généralement de l'ordre de 0,1 à 1,5, de préférence de 0,3 à 1,0.

Avantageusement, la réaction de déshydratation du glycérol peut être réalisée également en l'absence d'oxygène, mais en présence d'une quantité d'hydrogène allant de 0,1% à 10% en volume par rapport au mélange réactionnel, et d'un catalyseur choisi parmi ceux décrit dans la demande US 2008/018319.

La charge envoyée dans le réacteur peut être préalablement chauffée à une température de préchauffage de l'ordre d'environ 150°C à 350°C.

On opère à une pression de l'ordre de la pression atmosphérique et, plus précisément de préférence, à une pression légèrement supérieure.

La durée de contact exprimée en secondes est le rapport entre le volume du lit de catalyseur et le volume des réactifs gazeux envoyés par seconde. Les conditions moyennes de température et de pression existant dans un lit peuvent varier selon la nature du catalyseur, la nature du lit catalytique et la dimension du catalyseur. En général, la durée de contact est de 0,1 à 20 secondes et de préférence de 0,3 à 15 secondes.

À l'issue de l'étape (a), on obtient un flux aqueux, pouvant être liquide ou gazeux, contenant l'acroléine recherchée, de l'eau, du glycérol n'ayant pas réagi, et des sous-produits tels que hydroxypropanone, propanaldéhyde, acétaldéhyde, formaldéhyde, acide acrylique, acide propionique, acide acétique, acide formique, acétone, phénol, des produits d'addition de l'acroléine sur le glycérol, des produits de polycondensation du glycérol, des éthers de glycérol cycliques, ainsi que des de composés légers tels que azote, oxygène, monoxyde et dioxyde de carbone. Certains de ces produits sont des composés lourds, d'autres sont des composés légers condensables. Pour d'autres, il s'agit de composés légers incondensables dans les conditions de températures et de pression habituellement mises en oeuvre.

L'étape (b) dans le procédé selon l'invention consiste à purifier ledit flux aqueux pour obtenir un flux d'acroléine contenant moins de 15%, de préférence moins de 7%, de préférence encore moins de 4% massique d'eau par rapport à l'acroléine, c'est à dire un ratio massique eau/acroléine inférieur à 0,15, de préférence inférieur à 0,07, de préférence encore inférieur à 0,04.

Selon une première alternative, l'étape (b) consiste en une purification du flux contenant l'acroléine, comprenant une absorption dans de l'eau ou un flux aqueux recyclé pour laisser partir en tête les incondensables et récupérer en pied une solution aqueuse d'acroléine diluée, puis une séparation eau/acroléine par distillation pour obtenir en tête un flux gazeux ou liquide comprenant plus de 80% massique d'acroléine et moins de 15% massique d'eau par rapport à l'acroléine, de préférence plus de 90% massique d'acroléine et moins de 7% massique d'eau par rapport à l'acroléine et de préférence plus de 95% massique d'acroléine et moins de 4% massique d'eau par rapport à l'acroléine.

Le flux aqueux sortant de l'étape (a) peut être refroidi par un ou plusieurs échangeurs de chaleur.

Il est ensuite absorbé dans de l'eau ou un flux aqueux recyclé, pour former une solution aqueuse diluée d'acroléine, contenant typiquement moins de 7% massique d'acroléine. Cette absorption peut être réalisée dans une colonne à garnissage ou à plateau, de préférence à contre-courant. Avantageusement, on élimine en tête de la colonne les composés légers incondensables, tels que azote, oxygène, monoxyde et dioxyde de carbone.

La solution aqueuse d'acroléine est ensuite séparée par distillation. Pour cela, on peut utiliser un enchaînement de colonnes à distiller, comme décrit par exemple dans le brevet US 3,433,840 ou une seule colonne comme décrit par exemple dans les documents EP 1 300 384 ou EP 1 474 374. Cette distillation permet de récupérer, d'une part un flux constitué majoritairement d'eau, et d'autre part un flux gazeux ou liquide contenant une teneur massique en acroléine supérieure à 75% et une teneur massique en eau inférieure à 15% par rapport à l'acroléine et de préférence plus de 82% massique d'acroléine et moins de 7% d'eau par rapport à l'acroléine et de préférence plus de 95% massique d'acroléine et moins de 4% d'eau par rapport à l'acroléine. Ce dernier flux est envoyé vers l'étape (c).

Le flux constitué majoritairement d'eau contient également les impuretés lourdes, ayant un point d'ébullition plus élevé que celui de l'acroléine. La majeure partie de ce flux aqueux est recyclée à l'étape d'absorption et une petite partie de ce flux est éliminée, afin de déconcentrer la boucle d'eau en composés lourds. Les sous-produits légers, ayant un point d'ébullition plus faible que l'acroléine, comme par exemple l'acétaldéhyde, sont soit éliminés en tête dans la distillation, ou bien restent dans le flux d'acroléine envoyé vers l'étape (c) et sont éliminés lors d'une étape (d) de purification du MMP. Dans le cas où les sous-produits légers sont éliminés en tête de colonne de distillation, le flux gazeux ou liquide contenant une teneur massique en acroléine supérieure à 75% et une teneur massique en eau inférieure à 15% par rapport à l'acroléine, et de préférence plus de 82% massique d'acroléine et moins de 7% d'eau par rapport à l'acroléine et de préférence plus de 95% massique d'acroléine et moins de 4% d'eau par rapport à l'acroléine, peut être avantageusement récupéré par soutirage au niveau d'un plateau intermédiaire situé entre l'alimentation de la colonne et la tête de colonne, comme il est décrit par exemple dans le document EP 1 300 384.

Optionnellement, dans le cas où le flux aqueux sortant de l'étape (a) est gazeux, le refroidissement opéré sur le flux aqueux sortant de l'étape (a) peut être réalisé à une température telle que l'acroléine reste majoritairement sous forme gazeuse et qu'il se forme une phase liquide contenant une partie de l'eau et la majeure partie des composés ayant un point d'ébullition supérieur à l'eau, dont par exemple l'acide acrylique et le glycérol. Cette température est typiquement comprise entre 50°C et 100°C. La phase liquide est alors séparée de la phase gaz qui contient la majeure partie de l'acroléine. Cette étape de refroidissement peut aussi être réalisée dans une colonne garnie ou à plateaux. On peut injecter en tête de cette colonne un petit flux de liquide, par exemple une solution aqueuse. En pied de cette colonne, on récupère un flux de liquide qui contient les impuretés lourdes comme par exemple le glycérol et l'acide acrylique. En tête de cette colonne sort un flux gazeux aqueux contenant la majorité de l'acroléine. Le flux gazeux contenant l'acroléine est ensuite absorbé dans de l'eau comme décrit précédemment.

Selon une seconde alternative, l'étape (b) consiste en une purification partielle du flux aqueux gazeux contenant l'acroléine obtenu à l'étape (a), comprenant un refroidissement dudit flux gazeux et la séparation d'un flux liquide constitué de la majeure partie de l'eau et de l'acide acrylique, et un flux gazeux qui contient moins de 15% massique d'eau par rapport à l'acroléine et de préférence moins de 7% d'eau par rapport à l'acroléine et de préférence moins de 4% d'eau par rapport à l'acroléine. On peut, comme décrit dans le brevet EP 022 697, effectuer en premier un lavage du flux gazeux aqueux sortant de l'étape (a) avec une petite quantité d'eau dans une colonne de lavage pour éliminer l'acide acrylique, suivi d'un refroidissement du flux à une température allant généralement de 0°C à 50°C pour condenser la phase aqueuse, la majeure partie de l'acroléine restant sous forme gazeuse, puis un stripping de la phase aqueuse pour récupérer l'acroléine emportée dans l'eau.

On peut également utiliser une colonne de refroidissement telle que décrite dans le brevet EP 751 110 : le flux gazeux sortant de l'étape (a) est introduit en pied d'une colonne, de laquelle on soutire en pied un liquide dont la température est inférieure de quelques degrés au point de rosée du mélange gazeux de l'alimentation, lequel liquide est en partie refroidi puis injecté en tête de la colonne, l'autre partie du liquide est envoyé vers une colonne de strippage pour récupérer l'acroléine. Le flux gazeux obtenu en tête de la colonne de strippage est renvoyé dans la colonne de refroidissement avec le flux gazeux d'alimentation. Le flux gazeux sortant en tête de la colonne de refroidissement est. refroidi à une température généralement de 0°C à 50°C et les liquides condensés sont renvoyés en tête de la colonne de refroidissement.

Ces deux dispositifs permettent d'obtenir un mélange gazeux d'acroléine, de légers et de gaz incondensables, qui contient moins de 15% massique d'eau par rapport à l'acroléine et de préférence moins de 7% d'eau par rapport à l'acroléine et de préférence moins de 4% d'eau par rapport à l'acroléine. Dans la mesure où l'acroléine n'a pas été séparée des gaz incondensables ni des impuretés légères, la séparation des incondensables est effectuée à l'étape (c) et celle des impuretés légères est effectuée à l'étape (d).

Selon une troisième alternative, l'étape (b) consiste en une purification du flux d'acroléine sortant de l'étape (a) par distillation. Cette troisième alternative est avantageusement mise en oeuvre lorsque le flux sortant de l'étape (a) contient peu de gaz incondensables.

Le flux gazeux sortant de l'étape (a) est partiellement refroidi au moyen d'un ou plusieurs échangeurs de chaleur. Il est ensuite injecté dans une colonne à distiller qui comporte un condenseur qui permet de refroidir le flux sortant en tête de la colonne à distiller à une température de -10°C à 20°C sous une pression de 1 à 3 bars et un rebouilleur qui permet d'avoir une température de pied de 100°C à 130°C. Le taux de reflux de la colonne est avantageusement de 2 à 30. En pied on récupère un flux constitué d'eau et des impuretés lourdes. En sortie du condenseur de tête, on récupère une phase gaz constituée d'incondensables (azote, oxygène, dioxyde de carbone, monoxyde de carbone), qui est éliminée, et une phase liquide constituée à plus de 75% massique d'acroléine et moins de 15% massique d'eau par rapport à l'acroléine et de préférence plus de 82% massique d'acroléine et moins de 7% d'eau par rapport à l'acroléine et plus préférentiellement plus de 95% massique d'acroléine et moins de 4% d'eau par rapport à l'acroléine. Cette phase liquide est en majeure partie renvoyée dans la colonne pour faire le reflux et en partie soutirée pour alimenter l'étape (c). Alternativement, le condenseur de tête de la colonne à distiller peut fonctionner à une température plus élevée, de 30°C à 80°C : dans ce cas, le flux liquide sortant du condenseur est renvoyé dans la colonne pour faire le reflux et le flux gazeux, qui comporte l'acroléine et les incondensables, est refroidi dans second condenseur fonctionnant à une température de -10°C à +20°C de façon à récupérer une phase gaz constituée d'incondensables (azote, oxygène, dioxyde de carbone, monoxyde de carbone), qui est éliminée, et une phase liquide constituée à plus de 75% massique d'acroléine et moins de 15% massique d'eau par rapport à l'acroléine et de préférence plus de 82% massique d'acroléine et moins de 7% d'eau par rapport à l'acroléine et plus préférentiellement plus de 95% massique d'acroléine et moins de 4% d'eau par rapport à l'acroléine. Optionnellement la phase liquide peut subir une rectification complémentaire pour diminuer sa teneur en eau, et/ou un étêtage pour diminuer sa teneur en légers.

L'étape (c) dans le procédé selon l'invention consiste à faire réagir le flux d'acroléine liquide ou gazeux obtenu à l'étape (b) avec du méthylmercaptan en présence d'un catalyseur. La réaction est effectuée en phase liquide en présence d'un catalyseur basique, généralement en l'absence de solvant, à une température allant de préférence de 20°C à 60°C, avec un léger excès de MSH. Des catalyseurs utilisables sont par exemple des bases organiques, éventuellement combinées avec un acide organique. On peut citer par exemple les bases organiques sélectionnées parmi des amines telles que celles décrites dans la demande WO 96/40631, les bases organiques de type N-alkylmorpholine décrites dans le document EP 1 556 343, ou les systèmes constitués d'un composé basique et d'un composé acide dans des rapports molaires inférieurs à 0,3/1 décrits dans le document EP 1 408 029. De préférence, on utilise comme catalyseur la pyridine associée à l'acide acétique.

Cette réaction peut être mise en oeuvre de différentes façons, en batch ou en continu. L'acroléine liquide ou gazeuse et le méthylmercaptan sont introduits simultanément (temporellement ou spatialement) ou successivement dans le réacteur ou la colonne réactionnelle. Avantageusement, ils sont introduits sur un pied de MMP provenant d'un batch précédent ou sur un flux de MMP recyclé si l'on opère la réaction en continu. De nombreux procédés ont été décrits et peuvent être utilisés pour mettre en oeuvre cette étape (c).

Du fait de la purification du flux d'acroléine utilisé pour effectuer la réaction avec le méthylmercaptan, il est possible d'obtenir des rendements élevés en MMP, généralement supérieurs à ceux obtenus avec un procédé direct de fabrication de MMP à partir de glycérol.

Le MMP ainsi obtenu peut être utilisé tel quel ou purifié dans une étape (d), par exemple par distillation. On sépare ainsi des impuretés légères et des produits lourds soufrés qui sont ensuite incinérés.

La réaction du MMP, éventuellement purifié, avec l'acide cyanhydrique ou le cyanure de sodium s'effectue selon des conditions bien connues de l'homme du métier, selon les synthèses de Bücherer ou de Strecker, pour conduire, soit à la méthionine, soit à l'hydroxyanalogue de la méthionine, après transformation du produit de réaction, comme décrit dans le document « Techniques de l'Ingénieur, Traité Génie des procédés », J 6 410-1 à 9.

Selon un mode de réalisation préféré du procédé selon l'invention, l'étape (e) s'effectue par réaction du MMP avec de l'acide cyanhydrique en présence de carbonate d'ammonium pour former un composé hydantoïne, puis saponification avec de l'hydro-génocarbonate de potassium ou du carbonate de potassium, conduisant à du méthioninate de potassium, lequel, après acidification avec l'acide carbonique conduit à la D,L-méthionine qui cristallise.

Selon un autre mode de réalisation du procédé selon l'invention, l'étape (e) est réalisée par réaction du MMP avec HCN avec une catalyse pyridine ou une combinaison pyridine et acide acétique conduisant à un composé cyanhydrine, lequel est ensuite hydrolysé en milieu acide sulfurique en deux étapes successives mettant en oeuvre une première température basse (vers 50°C), et une seconde température plus élevée (vers 100°C). L'hydroxyanalogue de la méthionine est isolé ensuite soit par extraction, par exemple par un solvant tel que la méthylisobutylcétone ou la méthyléthylcétone, soit par acidification à l'acide sulfurique, suivie d'une décantation pour éliminer le sulfate d'ammonium, suivie d'une cristallisation de l'hydroxyanalogue de la méthionine.

La méthionine (ou l'hydroxyanalogue de la méthionine) obtenue suivant le procédé de l'invention est utilisée pour préparer des compositions destinées à l'alimentation de la volaille ou l'alimentation animale.

L'invention est décrite plus en détail par référence aux exemples suivants qui sont donnés à titre purement illustratif et nullement limitatif.

### EXEMPLES

### Exemple 1

Une solution aqueuse à 20% massique de glycérol (742 g/h) est vaporisée par passage dans un échangeur avec une température de peau de 350°C, puis injectée à pression atmosphérique conjointement à un flux d'air de débit 100 normaux litres par heure sur un réacteur tubulaire en lit fixe de 30 mm de diamètre intérieur, maintenu à 320°C et contenant 537 grammes de zircone tungstée (lot Z1044 Dai Ichi Kigenso).

Les gaz chauds absorbés sont envoyés vers une colonne d'absorption dans laquelle on injecte en tête 742 g/h d'eau avec une recirculation et comprenant un échangeur externe de telle façon que la température en tête de la colonne soit de 20°C. Le flux liquide contenant environ 5% d'acroléine est ensuite injecté dans une tour de distillation fonctionnant à pression atmosphérique. La phase vapeur est condensée et on récupère 87 g/h d'un liquide contenant environ 85% d'acroléine, 2,4% de formaldéhyde, 8% d'acétaldéhyde, 1,2% de propionaldéhyde, 0,2% d'acétone, 0,2% d'alcool allylique, et 3% d'eau.

Dans un réacteur de 500 mL, on introduit un pied de 50 g de MMP, puis 1,7 g d'un mélange de pyridine à 48% massique dans l'acide acétique. À une température de réaction de 60°C, on introduit simultanément sur une période de 30 minutes en agitant, 200 g de la solution d'acroléine à 85% obtenue ci-dessus, et 149,8 g de méthylmercaptan à 99,5%, puis on laisse réagir 10 minutes de plus à cette température, avant de rectifier le mélange sous pression réduite à 15 mm Hg. On récupère une fraction bouillant à environ 65°C de 353 g de MMP dont la pureté est estimée à 99%. L'acétaldéhyde, le propionaldéhyde, l'acétone et l'alcool allylique sont distillés à plus basse température. Le rendement chimique de transformation du glycérol en MMP s'élève à 78%. En utilisant du méthylmercaptan dérivé de biomasse, il est possible d'obtenir du MMP contenant 100% de carbone renouvelable.

### Exemple 2

Dans un réacteur fermé, on introduit 860 g d'une solution aqueuse de carbonate d'ammonium contenant 7,8% d'ammoniac et 12,4% de dioxyde de carbone. Après avoir chauffé à 90°C et sous agitation vigoureuse, on injecte progressivement 105 g du MMP produit à l'exemple 1 et 28,5 g d'acide cyanhydrique. On porte ensuite le mélange à 110°C pendant 1 heure. On refroidit à 80°C puis on dégaze lentement le réacteur de façon à ramener le mélange contenant la 5-(2-méthylmercaptoéthyl)hydantoïne à la pression atmosphérique. On ajoute 900 g d'une solution à 40% d'hydrogénocarbonate de potassium et on porte à 180°C sous 8 bars pendant 20 minutes. Le mélange est ensuite progressivement détendu à pression atmosphérique et une partie de l'eau est évaporée de façon à ce que le mélange contienne environ 20% de méthioninate de potassium. On refroidit alors aux alentours de 25°C et on injecte du dioxyde de carbone sous une pression de 2-3 bars de façon à abaisser le pH à 5,6. On obtient une suspension qui est filtrée. Le solide récupéré est lavé puis séché. On récupère 98 grammes de D,L-méthionine. Le filtrat est récupéré, concentré et peut être recyclé dans la solution contenant la 5-(2-méthylmercaptoéthyl)hydantoïne.

### Exemple 3

Dans un réacteur agité, on introduit 420 g de MMP produit à l'exemple 1, ainsi que 1,7 g d'un mélange de pyridine à 48% dans l'acide acétique. On injecte, sur une période de 45 minutes, 111 g d'acide cyanhydrique, tout en maintenant la température entre 35°C et 40°C, puis on chauffe pendant 30 minutes à 45°C. Le mélange obtenu, constitué principalement d'acide 2-hydroxy-4-(méthylthio)butyrique, est additionné sur une période de 45 minutes à 60°C à 591 g d'une solution aqueuse à 66% d'acide sulfurique. On laisse agiter 10 minutes à 65°C, puis on ajoute 380 g d'eau chaude à 80°C et on agite pendant 2 heures à 90°C. Le mélange réactionnel est ensuite extrait avec 1480 g de méthyl-isobutylcétone. La phase supérieure est ensuite évaporée à 70°C sous vide pour donner 637 g d'acide 2-hydroxy-4-(méthylthio)butyrique à 88% dans l'eau (hydroxyanalogue de la méthionine).

### Exemple 4

Une solution aqueuse à 33% de glycérol (450 g/h) est vaporisée par passage dans un échangeur avec une température de peau de 350°C, puis injectée à pression atmosphérique conjointement à un flux d'air de 100 normaux litres par heure et un flux de dioxyde de carbone de 50 normaux litres par heure sur un réacteur tubulaire en lit fixe de 30 mm de diamètre intérieur maintenu à 320°C et contenant 537 grammes de zircone tungstée (lot Z1044 Dai Ichi Kigenso)

Les gaz chauds sont envoyés en pied d'une colonne de refroidissement similaire à celle décrite dans le brevet EP 751 110, avec un refroidissement externe par recirculation. La température de pied est de 65°C. La solution aqueuse de pied est portée à 90°C et strippée par un léger flux d'azote de façon à obtenir en pied une solution aqueuse contenant 150 ppm d'acroléine. Les gaz issus de ce stripping (comprenant l'azote, l'acroléine et de l'eau) sont renvoyés vers la colonne de refroidissement.

Les gaz sortant de la tête de la colonne de refroidissement, comprenant 16% molaire d'acroléine, 1,5% molaire d'eau (soit un ratio massique eau/acroléine de 0,035) dans un mélange d'azote, CO₂, CO, acétaldéhyde et O₂ sont envoyés en pied d'une colonne d'absorption alimentée en tête par du MMP refroidi à -10°C. Le pied de la colonne est envoyé dans un réacteur agité continu, dans lequel on injecte 64,5 g/h de méthylmercaptan et 0,8 g/h d'un mélange de pyridine à 48% massique dans l'acide acétique. La réaction est réalisée dans la phase liquide. Le MMP produit est rectifié sous pression réduite à 15 mm Hg et on récupère une fraction bouillant à environ 65°C.

Le rendement chimique de transformation du glycérol en MMP s'élève à 76%.

### Exemple 5

Une solution aqueuse à 25% de glycérol (742 g/h) est vaporisée par passage dans un échangeur avec une température de peau de 350°C, puis injectée sous 2,8 bars absolus conjointement à un flux d'oxygène de 10 normaux litres par heure sur un réacteur tubulaire en lit fixe de 30 mm de diamètre intérieur maintenu à 320°C et contenant 500 grammes de zircone tungstée (lot Z1044 Dai Ichi Kigenso).

Les gaz chauds sortant du réacteur sont refroidis à 150°C puis envoyés au tiers supérieur de la hauteur d'une colonne à distiller de 10 plateaux qui comporte en tête un condenseur refroidissant à 67°C sous 2 bars absolus. En pied de cette colonne, on récupère un flux de 630 g/h contenant l'eau et les impuretés lourdes. En sortie du condenseur de tête, on récupère une phase liquide (1540 g/h) que l'on réinjecte totalement dans la colonne, et un flux gazeux. Ledit flux gazeux est refroidi à 5°C via un deuxième condenseur, qui permet de laisser échapper les incondensables (CO, CO₂ et O₂) et de récupérer 108 g/h d'un liquide contenant 83% d'acroléine et 5,5% d'eau.

Ce flux d'acroléine, ainsi qu'un flux de 80 g/h de méthylmercaptan et 0,8 g/h d'un mélange de pyridine à 10% massique dans l'acide acétique sont injectés dans une colonne garnie refroidie à 30°C et muni d'une recirculation d'une partie du liquide sortant en pied vers la tête. L'autre partie du liquide sortant en pied est ensuite étêté, puis rectifié sous pression réduite à 15 mm Hg et on récupère une fraction bouillant à environ 65°C, constitué de MMP ayant une pureté de 98%.

Le rendement chimique de transformation du glycérol en MMP s'élève à 75%.

### Exemple 6 (comparatif)

Une solution aqueuse à 20% massique de glycérol (742 g/h) est vaporisée par passage dans un échangeur avec une température de peau de 350°C, puis injectée à pression atmosphérique conjointement à un flux d'air de débit 100 normaux litres par heure sur un réacteur tubulaire en lit fixe de 30 mm de diamètre intérieur, maintenu à 320°C et contenant 537 grammes de zircone tungstée (lot Z1044 Dai Ichi Kigenso).

Les gaz chauds absorbés sont envoyés vers une colonne d'absorption dans laquelle on injecte en tête 742 g/h d'eau avec une recirculation et comprenant un échangeur externe de telle façon que la température en tête de la colonne soit de 20°C. Le flux liquide contenant environ 5% d'acroléine est ensuite injecté dans une tour de distillation fonctionnant à pression atmosphérique comportant moins de plateaux qu'à l'exemple 1. La phase vapeur est condensée et on récupère 101 g/h d'un liquide contenant environ 73,5% d'acroléine, 2,0% de formaldéhyde, 6,9% d'acétaldéhyde, 1,0% de propionaldéhyde, 0,2% d'acétone, 0,2% d'alcool allylique, et 16,2% d'eau, soit une teneur en eau de 22% massique par rapport à l'acroléine.

Dans un réacteur de 500 mL, on introduit un pied de 50 g de MMP, puis 1,7 g d'un mélange de pyridine à 48% massique dans l'acide acétique. À une température de réaction de 60°C, on introduit simultanément sur une période de 30 minutes en agitant, 200 g de la solution d'acroléine à 73,5% obtenue ci-dessus, et 139 g de méthylmercaptan à 99,5%, puis on laisse réagir 10 minutes de plus à cette température, avant de rectifier le mélange sous pression réduite à 15 mm Hg. On récupère une fraction bouillant à environ 65°C de 161 g de MMP dont la pureté est estimée à 98%. Le rendement chimique de transformation du glycérol en MMP s'élève à 33%.

## Revendications

1. Procédé de fabrication du méthylmercaptopropionaldéhyde par addition de méthylmercaptan sur l'acroléine, **caractérisé par le fait qu'**au moins l'un parmi l'acroléine et le méthylmercaptan mis en jeu dans cette réaction a été obtenu par une réaction ou une succession de réactions à partir de la biomasse, ledit procédé comprenant au moins les étapes suivantes :
(a) la déshydratation de glycérol en acroléine à partir d'une solution aqueuse de glycérol en présence d'un catalyseur acide,
(b) la purification du flux aqueux issu de l'étape (a) pour obtenir un flux d'acroléine contenant moins de 15%, de préférence moins de 7%, de préférence encore moins de 4% massique d'eau par rapport à l'acroléine,
(c) la réaction du flux d'acroléine obtenu à l'étape (b) avec du méthylmercaptan en présence d'un catalyseur,
(d) éventuellement la purification du produit obtenu à l'étape (c).

2. Procédé de fabrication de la méthionine ou de l'hydroxyanalogue de la méthionine par réaction de l'acide cyanhydrique ou du sel de sodium de l'acide cyanhydrique sur le méthylmercaptopropionaldéhyde, le méthylmercaptopropionaldéhyde ayant été obtenu par addition de méthylmercaptan sur l'acroléine, **caractérisé par le fait qu'**au moins l'un parmi l'acroléine, le méthylmercaptan ou l'acide cyanhydrique mis en jeu a été obtenu par une réaction ou une succession de réactions à partir de la biomasse, ledit procédé comprenant au moins les étapes suivantes :
(a) la déshydratation de glycérol en acroléine à partir d'une solution aqueuse de glycérol en présence d'un catalyseur acide,
(b) la purification du flux aqueux issu de l'étape (a) pour obtenir un flux d'acroléine contenant moins de 15%, de préférence moins de 7%, de préférence encore moins de 4% massique d'eau par rapport à l'acroléine,
(c) la réaction du flux d'acroléine obtenu à l'étape (b) avec du méthylmercaptan en présence d'un catalyseur,
(d) éventuellement, la purification du produit obtenu à l'étape (c),
(e) la réaction du produit obtenu à l'étape (c) ou (d) avec de l'acide cyanhydrique, ou du cyanure de sodium,
(f) la transformation du produit obtenu en (e) en méthionine ou hydroxyanalogue de la méthionine et sa purification.

3. Procédé selon la revendication 1 ou 2 **caractérisé en ce que** l'on a obtenu l'acroléine par déshydratation de glycérol.

4. Procédé selon l'une quelconque des revendications 1 à 3 **caractérisé en ce que** l'étape (b) comprend une absorption dans de l'eau ou un flux aqueux recyclé, pour laisser partir en tête les incondensables et récupérer en pied une solution aqueuse d'acroléine diluée, puis une séparation eau/acroléine par distillation pour obtenir en tête un flux gazeux ou liquide comprenant plus de 80% massique d'acroléine et moins de 15% massique d'eau par rapport à l'acroléine.

5. Procédé selon l'une quelconque des revendications 1 à 3 **caractérisé en ce que** l'étape (b) consiste en une purification partielle du flux aqueux gazeux contenant l'acroléine obtenu à l'étape (a), comprenant un refroidissement dudit flux gazeux et la séparation d'un flux liquide constitué de la majeure partie de l'eau et de l'acide acrylique, et un flux gazeux qui contient moins de 15%, de préférence moins de 7%, de préférence encore moins de 4% massique d'eau par rapport à l'acroléine.

6. Procédé selon l'une quelconque des revendications 1 à 3 **caractérisé en ce que** l'étape (b) consiste en une purification du flux d'acroléine sortant de l'étape (a) par distillation.

7. Procédé selon l'une quelconque des revendications 1 à 6 **caractérisé en ce que** l'étape (c) est réalisée en phase liquide en présence d'un catalyseur basique, en l'absence de solvant, à une température allant de préférence de 20°C à 60°C, avec un léger excès de méthanethiol.

## Patentansprüche

1. Verfahren zur Herstellung von Methylmercaptopropionaldehyd durch Addition von Methylmercaptan an Acrolein, **dadurch gekennzeichnet, dass** das Acrolein und/oder das Methylmercaptan, das bei dieser Reaktion eingesetzt wird, durch eine von Biomasse ausgehende Reaktion oder Abfolge von Reaktionen erhalten wurde, wobei das Verfahren mindestens die folgenden Schritte umfasst:
(a) Dehydratisierung von Glycerin zu Acrolein ausgehend von einer wässrigen Lösung von Glycerin in Gegenwart eines sauren Katalysators,
(b) Reinigung des wässrigen Stroms aus Schritt (a) zum Erhalt eines Acroleinstroms, der weniger als 15 Gew.-%, vorzugsweise weniger als 7 Gew.-% und weiter bevorzugt weniger als 4 Gew.-% Wasser, bezogen auf das Acrolein, enthält,
(c) Umsetzung des Acroleinstroms aus Schritt (b) mit Methylmercaptan in Gegenwart eines Katalysators,
(d) gegebenenfalls Reinigung des Produkts aus Schritt (c).

2. Verfahren zur Herstellung von Methionin oder dem Hydroxy-Analog von Methionin durch Umsetzung von Cyanwasserstoffsäure oder dem Natriumsalz von Cyanwasserstoffsäure mit Methylmercaptopropionaldehyd, wobei der Methylmercaptopropionaldehyd durch Addition von Methylmercaptan an Acrolein erhalten wurde, **dadurch gekennzeichnet, dass** das eingesetzte Acrolein und/oder das eingesetzte Methylmercaptan und/oder die eingesetzte Cyanwasserstoffsäure durch eine von Biomasse ausgehende Reaktion oder Abfolge von Reaktionen erhalten wurde, wobei das Verfahren mindestens die folgenden Schritte umfasst:
(a) Dehydratisierung von Glycerin zu Acrolein ausgehend von einer wässrigen Lösung von Glycerin in Gegenwart eines sauren Katalysators,
(b) Reinigung des wässrigen Stroms aus Schritt (a) zum Erhalt eines Acroleinstroms, der weniger als 15 Gew.-%, vorzugsweise weniger als 7 Gew.-% und weiter bevorzugt weniger als 4 Gew.-% Wasser, bezogen auf das Acrolein, enthält,
(c) Umsetzung des Acroleinstroms aus Schritt (b) mit Methylmercaptan in Gegenwart eines Katalysators,
(d) gegebenenfalls Reinigung des Produkts aus Schritt (c),
(e) Umsetzung des Produkts aus Schritt (c) oder (d) mit Cyanwasserstoffsäure oder Natriumcyanid,
(f) Umwandlung des Produkts aus (e) in Methionin oder das Hydroxy-Analog von Methionin und Reinigung davon.

3. Verfahren nach Anspruch 1 oder 2, **dadurch gekennzeichnet, dass** das Acrolein durch Dehydratisierung von Glycerin erhalten wurde.

4. Verfahren nach einem der Ansprüche 1 bis 3, **dadurch gekennzeichnet, dass** der Schritt (b) eine Absorption in Wasser oder einem rezyklierten wässrigen Strom zum Austretenlassen von nicht kondensierbaren Substanzen am Kopf und zur Gewinnung einer verdünnten wässrigen Lösung von Acrolein am Sumpf und eine anschließende Wasser/Acrolein-Trennung durch Destillation zum Erhalt eines gasförmigen oder flüssigen Stroms, der mehr als 80 Gew.-% Acrolein und weniger als 15 Gew.-% Wasser, bezogen auf das Acrolein, umfasst, am Kopf, umfasst.

5. Verfahren nach einem der Ansprüche 1 bis 3, **dadurch gekennzeichnet, dass** der Schritt (b) aus einer teilweisen Reinigung des Acrolein enthaltenden gasförmigen wässrigen Stroms aus Schritt (a) besteht, der eine Abkühlung des gasförmigen Stroms und die Abtrennung eines aus dem größten Teil des Wassers und der Acrylsäure bestehenden flüssigen Stroms und eines gasförmigen Stroms, der weniger als 15 Gew.-%, vorzugsweise weniger als 7 Gew.-% und weiter bevorzugt weniger als 4 Gew.-% Wasser, bezogen auf das Acrolein, enthält, umfasst.

6. Verfahren nach einem der Ansprüche 1 bis 3, **dadurch gekennzeichnet, dass** der Schritt (b) aus einer Reinigung des Acroleinstroms aus Schritt (a) durch Destillation umfasst.

7. Verfahren nach einem der Ansprüche 1 bis 6, **dadurch gekennzeichnet, dass** der Schritt (c) in flüssiger Phase in Gegenwart eines basischen Katalysators in Abwesenheit von Lösungsmitteln bei einer Temperatur, die vorzugsweise im Bereich von 20 °C bis 60 °C liegt, mit einem leichten Überschuss von Methanthiol durchgeführt wird.

## Claims

1. Process for manufacturing methylmercaptopropionaldehyde by adding methyl mercaptan to acrolein, **characterized in that** at least one from among the acrolein and the methyl mercaptan employed in this reaction is obtained via a reaction or a sequence of reactions starting from biomass, said process comprising at least the following steps:
(a) dehydration of glycerol to acrolein using an aqueous glycerol solution in the presence of an acidic catalyst,
(b) purification of the aqueous stream obtained from step (a) to obtain an acrolein stream containing less than 15%, preferably less than 7% and more preferably less than 4% by mass of water relative to the acrolein,
(c) reaction of the acrolein stream obtained in step (b) with methyl mercaptan in the presence of a catalyst,
(d) optionally, purification of the product obtained in step (c).

2. Process for manufacturing methionine or the hydroxy analogue of methionine by reacting hydrogen cyanide or the sodium salt of hydrogen cyanide with methylmercaptopropionaldehyde, the methylmercaptopropionaldehyde having been obtained by adding methyl mercaptan to acrolein, **characterized in that** at least one from among the acrolein, the methyl mercaptan and the hydrogen cyanide employed is obtained via a reaction or a sequence of reactions starting from biomass, said process comprising at least the following steps:
(a) dehydration of glycerol to acrolein using an aqueous glycerol solution in the presence of an acidic catalyst,
(b) purification of the aqueous stream obtained from step (a) to obtain an acrolein stream containing less than 15%, preferably less than 7% and more preferably less than 4% by mass of water relative to the acrolein,
(c) reaction of the acrolein stream obtained in step (b) with methyl mercaptan in the presence of a catalyst,
(d) optionally, purification of the product obtained in step (c),
(e) reaction of the product obtained in step (c) or (d) with hydrogen cyanide or sodium cyanide,
(f) conversion of the product obtained in (e) into methionine or the hydroxy analogue of methionine, and purification thereof.

3. Process according to Claim 1 or 2, **characterized in that** the acrolein is obtained by dehydrating glycerol.

4. Process according to any one of Claims 1 to 3, **characterized in that** step (b) comprises an absorption in water or a recycled aqueous stream, to allow departure as a head fraction of the uncondensables and to recover as a tail fraction a dilute aqueous acrolein solution, followed by a water/acrolein separation by distillation to obtain as the head fraction a gaseous or liquid stream comprising more than 80% by mass of acrolein and less than 15% by mass of water relative to the acrolein.

5. Process according to any one of Claims 1 to 3, **characterized in that** step (b) consists in partially purifying the gaseous aqueous stream containing acrolein obtained in step (a), comprising cooling of the said gaseous stream and separation of a liquid stream formed from the majority of the water and acrylic acid, and a gaseous stream that contains less than 15%, preferably less than 7% and more preferably less than 4% by mass water relative to the acrolein.

6. Process according to any one of Claims 1 to 3, **characterized in that** step (b) consists in purifying the acrolein stream exiting step (a) by distillation.

7. Process according to any one of Claims 1 to 6, **characterized in that** step (c) is performed in the liquid phase in the presence of a basic catalyst, in the absence of solvent, at a temperature preferably ranging from 20°C to 60°C, with a slight excess of methanethiol.
